Europäisches Patentamt

European Patent Office

Office européen des brevets

(11) Veröffentlichungsnummer: **0 033 080**
**A2**

(12) **EUROPÄISCHE PATENTANMELDUNG**

(21) Anmeldenummer: **81100171.8**

(22) Anmeldetag: **13.01.81**

(51) Int. Cl.³: **A 61 M 1/03**

(30) Priorität: **23.01.80 DE 8001665 U**

(43) Veröffentlichungstag der Anmeldung:
.05.08.81 **Patentblatt 81/31**

(84) Benannte Vertragsstaaten:
**AT BE CH DE FR GB IT LI LU NL SE**

(71) Anmelder: **ASID BONZ & SOHN GMBH**
**Feldstrasse 1a**
**D-8044 Unterschleissheim(DE)**

(72) Erfinder: **Brauner, Holger, Dr.**
**Uttstädterstrasse 1**
**D-8551 Adelsdorf-Aisch(DE)**

(72) Erfinder: **Steinhauer, Jürgen**
**Orffstrasse 29**
**D-7114 Pfedelbach(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) Dialyseschlauchsystem.

(57) Dialyseschlauchsystem bestehend aus weichmacherarmen Kunststoffschläuchen (2) und (3) und Kunststoff-Verbindungsstücken (1), (7) und (8), wobei die Verbindungsstücke zur Aufnahme der Schläuche dem äußeren Schlauchdurchmesser angepaßte weite Bohrungen (4) und (5) aufweisen, an die sich dem inneren Schlauchdurchmesser angepaßte enge Bohrungen (6) anschließen.

EP 0 033 080 A2

./...

FIG. 11

0033080

ASID BONZ & SOHN GMBH     HOE 80/S 001          Dr. MD/cr

## Dialyseschlauchsystem

Gegenstand der Erfindung ist ein Dialyseschlauchsystem, wie es zum Anschluß nierenkranker Patienten
an künstliche Nieren benötigt wird. Es zeichnet sich
durch eine besonders zweckmäßige Konstruktion der Verbindungsstücke aus, mit denen die verschiedenartigen
Schläuche, die für ein derartiges Schlauchsystem erforderlich sind, aneinander gekoppelt werden.

Ein Dialyseschlauchsystem besteht auf der arteriellen
Seite, auf der dem Patienten das Blut abgenommen wird,
mindestens aus einem mit Fistelanschluß und Schutzkappe versehenen Blutschlauch, aus einem Heparindosierungsanschluß und einem Pumpschlauch und auf der
venösen Seite aus einem mit einem Fistelschlauch und
Schutzkappe verschließbaren Blutschlauch, einer Tropfkammer mit einem Filter und einem Druckmonitoranschluß.
Darüberhinaus sind jedoch in der Regel weitere Anschlußelemente erforderlich, die an verschiedenen
Stellen des Schlauchsystems die Entnahme von Blutproben bzw. die Zuführung weiterer Substanzen zum Blut
ermöglichen oder den Anschluß zusätzlicher Meßinstrumente
erlauben sollen. Das hat dazu geführt, daß bei herkömmlichen Dialyseschlauchsystemen eine Vielzahl von
Schläuchen mit unterschiedlichen Durchmessern miteinander verbunden werden müssen.

Den rheologischen Problemen, die im Dialyseschlauchsystem beim Pumpen des Blutes durch Schläuche unterschiedlichen Durchmessers auftreten, hat man bisher wenig
Beachtung geschenkt. Insbesondere ist vernachlässigt
worden, daß beim abrupten Übergang des Blutstromes
an den Verbindungsstellen von Schläuchen unterschiedlichen Durchmessers die Blutbestandteile erheblichen mechanischen Belastungen ausgesetzt sind, die zu einer Schädigung des Blutes führen können. Darüberhinaus fehlt es bei den bekannten Dialyseschläuchen an der Möglichkeit, die erforderlichen Zusatzgeräte in einwandfreier Weise an das Schlauchsystem anzuschließen.

Es stellte sich deshalb die Aufgabe, Verbindungsmöglichkeiten für die bei der Dialyse erforderlichen Schläuche zu schaffen, die einen naht- und fugenlosen Übergang zwischen Schläuchen unterschiedlichen Durchmessers gewährleisten. Diese Verbindungsstücke müssen so beschaffen sein, daß sie auch den Anschluß etwaiger Zusatzgeräte ermöglichen und die Entnahme von Blut oder die Zuführung von Medikamenten oder anderen Substanzen ermöglichen. Es ist selbstverständlich, daß dabei alle mit dem Blut in Kontakt kommenden Teile aus einem Material bestehen müssen, aus dem sich keine das Blut chemisch schädigenden Bestandteile herauslösen können.

Die vorstehend genannten Aufgaben werden durch ein Dialyseschlauchsystem gelöst, das aus weichmacherarmen Kunststoffschläuchen und -verbindungsstücken besteht, wobei die Verbindungsstücke zur Aufnahme der Schläuche dem äußeren Schlauchdurchmesser angepaßte weite Bohrungen aufweisen, an die sich dem inneren Schlauchdurchmesser angepaßte enge Bohrungen anschließen.

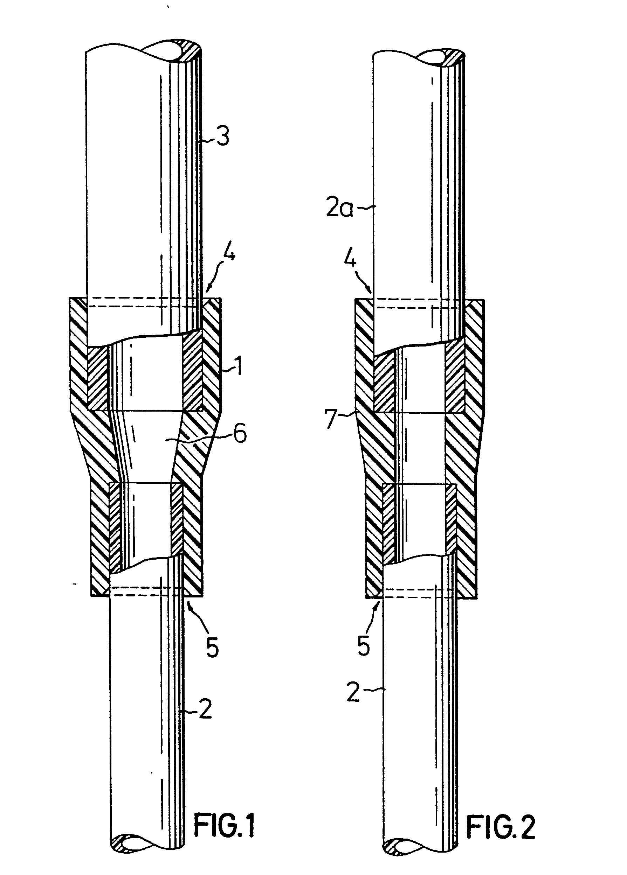

Figur 1 zeigt ein derartiges Verbindungsstück (1), mit dem Schläuche (2) und (3) verschiedenen Außen- und Innendurchmessers verbunden werden. Die beiden Schlauchtypen werden in die jeweiligen weiten, ihrem äußeren Durchmesser angepaßten Bohrungen (4) und (5) bis zum Anstoß an die enge Innenbohrung (6) eingeschoben und mit dem Verbindungsstück dauerhaft verbunden. Durch eine gleichmäßige Abschrägung der inneren Bohrung (6) des Verbindungsstückes (1) wird ein fließender Übergang des Blutstromes von einem Schlauch in den anderen erreicht.

Figur 2 zeigt ein entsprechendes Verbindungsstück (7), mit dem Schläuche (2) und (2a) verbunden werden, bei denen nur die Außen-, nicht aber die Innendurchmesser unterschiedlich sind, so daß der Blutstrom hier, ohne auf Widerstand zu stoßen, gleichmäßig durchfließen kann.

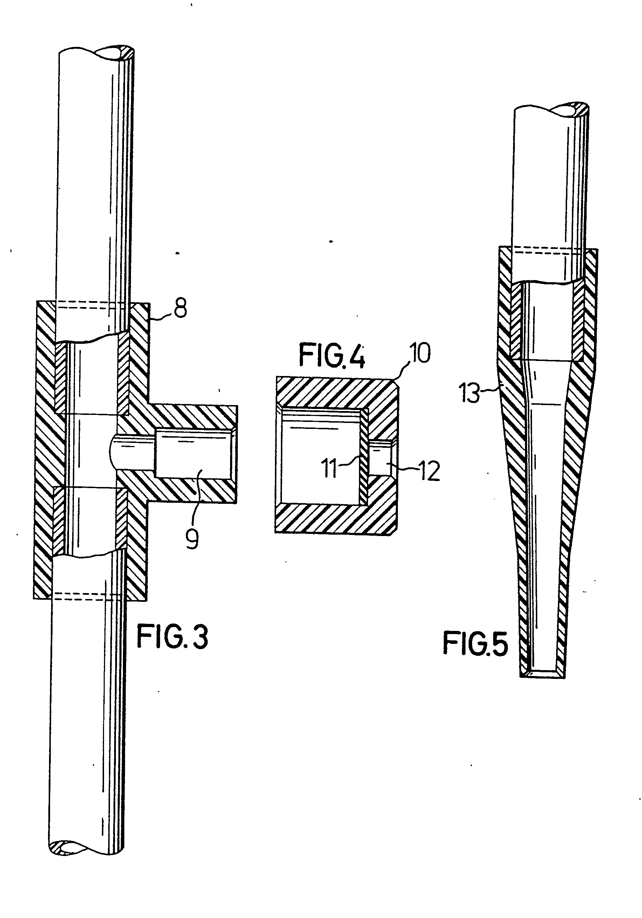

Figur 3 zeigt ein Verbindungsstück (8) in T-form, durch das ein Zusatzgerät mit engerem Schlauchdurchmesser bei (9) angeschlossen werden kann. Auch hier weist die innere Bohrung des Verbindungsstückes den gleichen Durchmesser auf, wie der Innendurchmesser der jeweils anzuschließenden Schläuche. Selbstverständlich kann die Anschlußstelle auch so gestaltet werden, daß die Verbindung mit einem Schlauch dickeren Durchmessers möglich ist.

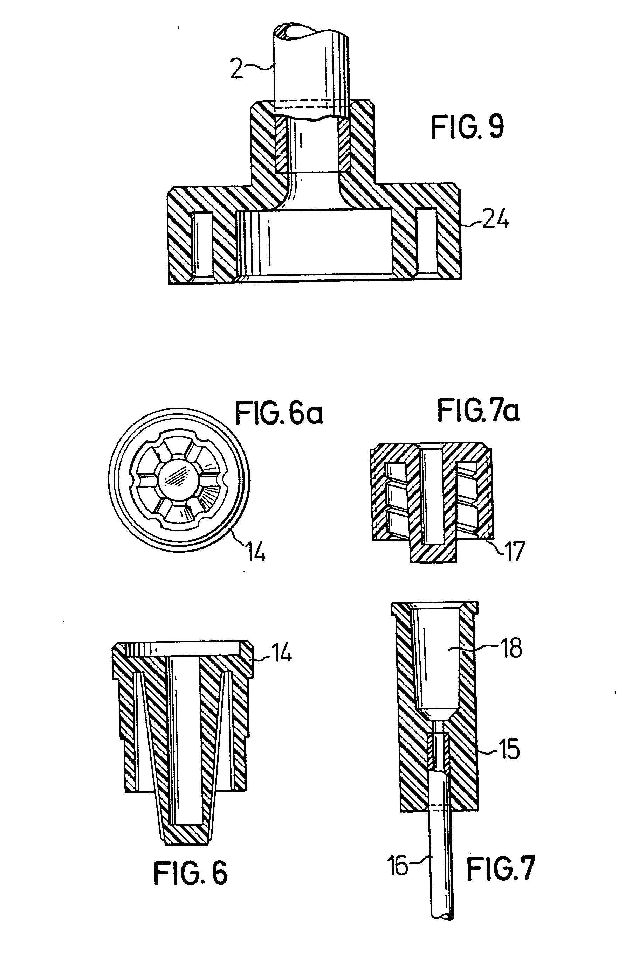

Figur 4 zeigt eine Verschlußkappe (10) für die Abzweigung (9) des T-Stückes von Figur 3, in die eine Latexmembran (11) eingelegt wird. Durch die Aussparung (12) in der Verschlußkappe (10) kann die Latexmembran mit einer Injektionsnadel durchstochen und so Proben entnommen oder zusätzliche Stoffe in den Blutstrom eingegeben werden. Die vorstehend dargelegte Art der Verbindung zwischen Schlauch und Verbindungsstück zeigt auch der in Fig. 5 dargestellte Fistelanschluß (13). Auf ihn kann eine Injektionskanüle aufgesetzt werden. Bis zum Gebrauch und nach Beendigung der Dialyse wird der Fistelanschluß mit einer Schutzkappe verschlossen, die dort nicht gezeichnet ist.

Eine belüftete Verschlußkappe (14), die auf das Schlauchsystem zur Durchführung der Ethylenoxidsterilisation aufgesteckt werden muß, ist in Figur 6 im Schnitt dargestellt. Figur 6a zeigt die belüftete Verschlußkappe (14) von unten.

Im allgemeinen werden nicht benutzte Schlauchanschlüsse durch Luer-Lok-Verschlüsse, wie sie in Fig. 7 und 7a gezeigt werden, geschlossen. Das Verschlußstück (15) weist dabei die zur Aufnahme des Schlauches (16) geeigneten, vorstehend beschriebenen Bohrungen auf, die einen gleitenden Übergang der Flüssigkeit vom Schlauch in den Verschluß ermöglichen. Durch Einsetzen des Deckels (17) in den Innenkegel (18) kann der Zugang zum Schlauchsystem abgedichtet werden.

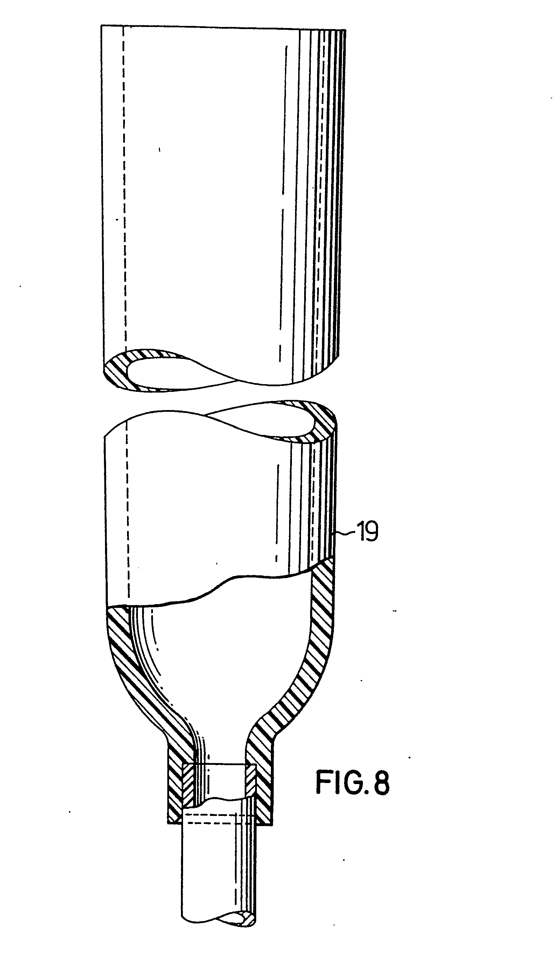

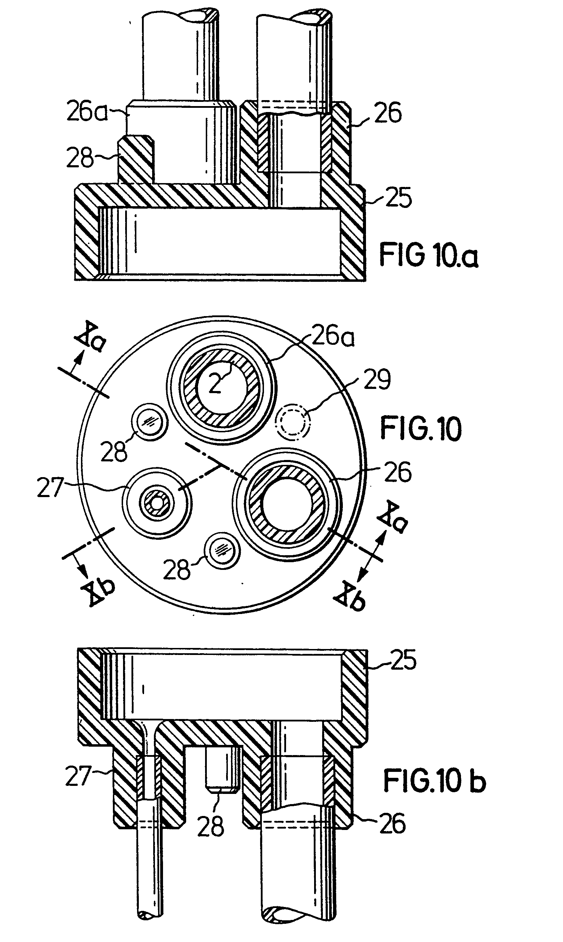

Die nach dem geschilderten Konstruktionsprinzip gestalteten Verbindungsstücke können auch als Deckel für ein aus durchsichtigem Kunststoff geblasenes Hohlgefäß (19) (Figur 8) geformt werden. Dieses Hohlgefäß kann entweder als Luftfänger (20), als venöse Tropfkammer (21) oder als Behälter (22) für einen Druckübertragungsballon (23) dienen. Die jeweilige Funktion des Behälters im Dialyseschlauchsystem wird durch die Deckelkonstruktion bestimmt. Ein nur einen Schlauchanschluß (2) aufweisender Deckel (24) (Figur 9) wird bei Verwendung des Hohlgefäßes zur Aufnahme des Druckübertragungsballons (23) gewählt. Soll der Behälter als Tropfkammer (21) oder Luftfänger (20) dienen, dann wird ein Deckel (25) mit mehreren Schlauchanschlüsse (26), (26a) und (27) (Figur 10) verwendet, die jeweils die für die erfindungsgemäßen Verbindungsstücke typische Anordnung von innerer und äußerer Bohrung aufweisen. Die Figuren 10a und 10b zeigen Schnitte durch den in Fig. 10 in Draufsicht dargestellten Deckel in den Ebenen Xa - Xa und Xb - Xb. An dem Deckel sind weiterhin zwei Zapfen (28) angebracht, die zur Ausbildung einer Aufhängevorrichtung dienen können, sowie eine Bedarfsbohrung (29), die für weitere Schlauchzugänge verwendet werden kann. Mit Hilfe einer Latexmembran lassen sich an den zusätzlichen Anschlüssen eines derartigen Deckels Injektionszugänge herstellen, um dem Patienten Blut zu entnehmen, dessen korpuskuläre Bestandteile sedimentiert und dann dem Patienten zurückgegeben werden. Die erforderlichen Untersuchungen werden dann direkt aus dem Serum vorgenommen.

In die venöse Tropfkammer wird zweckmäßig ein stehendes
Filter (31) aus Polyamid oder Polyäthylen eingesetzt,
dessen Maschenweite die Entfernung größerer Fremdkörper aus dem Blut erlaubt.

Als Material für die Schläuche kommen vor allem weichmacherarmes, für medizinische Zwecke zugelassenes
Chlorpolyäthylen oder PVC, die mit Äthylenoxyd
sterilisiert werden können, in Betracht. Die gleichen
Materialien , zusätzlich aber auch lineare Copolyätherurethane können zur Herstellung der Verbindungsstücke verwendet werden.

Eine dauerhafte Verbindung der Schläuche mit den
Verbindungsstücken wird zweckmäßigerweise durch das
Lösungsmittelschweißen erreicht. Dabei wird die dem
äußeren Schlauchdurchmesser angepaßte weite Bohrung
des Verbindungsstückes innen mit einem geeigneten Lösungsmittel wie Dimethylformamid oder Tetrahydrofuran,
das bis zu 25 % gelöstes Polyvinylchlorid enthalten
kann, befeuchtet und im feuchten Zustand der passende
Schlauch eingeschoben.

Das so hergestellte Dialyseschlauchsystem stellt sicher,
daß mechanische Schädigungen des Blutes während des
Transportes durch Dialyseschläuche vermieden werden.
Es ist einfach zusammenzusetzen und bietet einwandfreie Anschlußmöglichkeiten für Zusatzgeräte. Darüberhinaus ist die Entnahme von Blutproben oder die Zuführung von Medikamenten oder anderen Substanzen auf
einfache Weise möglich.

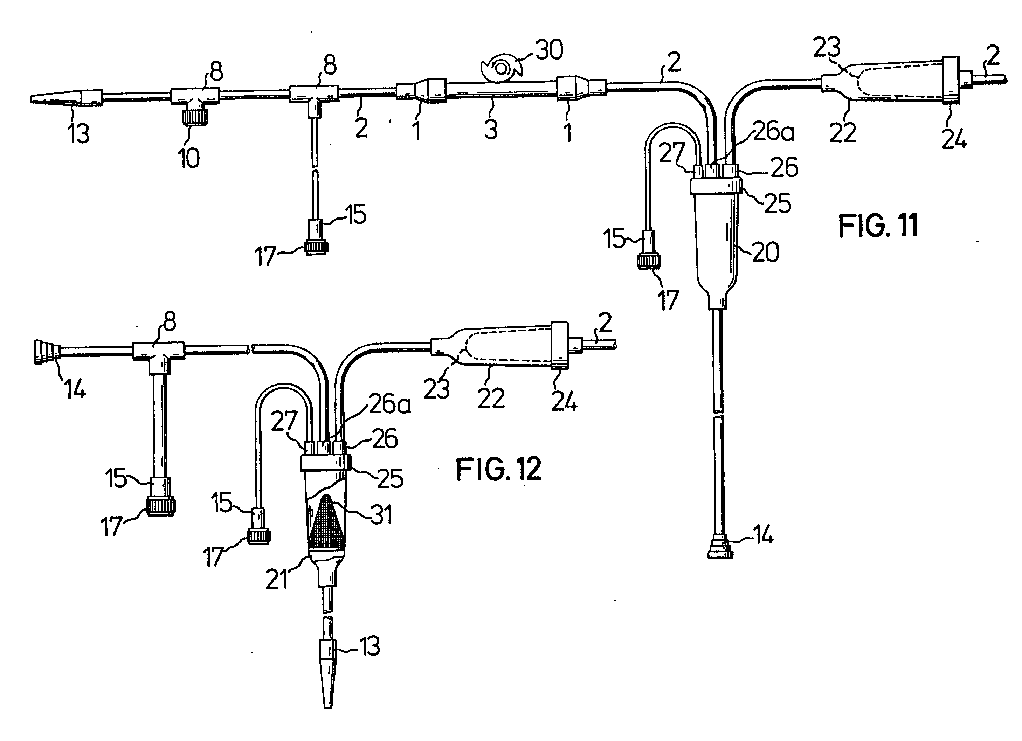

Figuren 11 und 12 zeigen eine Möglichkeit, wie ein Dialyseschlauchsystem ein mit Hilfe der geschriebenen Verbindungsstücke zusammengefügt werden kann. Selbstverständlich können die einzelnen Elemente auch in anderer Weise zusammengesetzt werden, je nachdem welche Anschlüsse und Zusatzgeräte benötigt werden. Es ist der besondere Vorteil des erfindungsgemäßen Dialyseschlauchsystems, daß es die Möglichkeit gibt, mit wenigen einfachen Elementen das Schlauchsystem in der verschiedenartigsten Weise zusammenzufügen und es damit den jeweiligen Anforderungen der Praxis optimal anzupassen.

Figur 11 stellt den arteriellen Teil des Dialyseschlauchsystems dar. Das dem Patienten entnommene Blut tritt über eine nicht gezeichnete Kanüle und den Fistelanschluß (13) in das Schlauchsystem ein. Über Anschlußstücke (8), mit denen Zusatzgeräte oder Injektions- bzw. Blutabnahmemöglichkeiten geschaffen werden, erreicht der Blutstrom das Schlauchstück (3), an dem eine Pumpvorrichtung (30) für den Weitertransport des Blutes sorgt. Die durch Verbindungsstücke (1) oder (7) aneinander gekuppelten Schläuche führen das Blut zum Luftfänger (20), der über den Deckel Verbindungen zur Druckkammer (22) und anderen Anschlußgeräten erlaubt. Über das belüftete Ventil (14) erreicht der Blutstrom dann den Dialysator.

Nach der Reinigung des Blutes im Dialysator fließt das Blut im venösen Teil des Dialyseschlauchsystems weiter, wie es beispielsweise in Fig. 12 dargestellt ist. Auch hier besteht die Möglichkeit, durch den Einsatz der Verbindungsstücke vielfältige Anschlußmöglichkeiten zu schaffen. Schließlich erreicht das Blut die venöse Tropfkammer (21), in der das Blut über ein stehendes Filter (31) läuft. Danach kann das Blut über den Fistelanschluß (13) wieder dem Patienten zugeführt werden.

**0033080**

**Patentansprüche:**

1. Dialyseschlauchsystem, dadurch gekennzeichnet, daß es aus weichmacherarmen Kunststoffschläuchen (2) und (3) und Kunststoffverbindungsstücken (1), (7) und (8) besteht, wobei die Verbindungsstücke zur Aufnahme der Schläuche dem äußeren Schlauchdurchmesser angepaßte weite Bohrungen (4) und (5) aufweisen, an die sich dem inneren Schlauchdurchmesser angepaßte enge Bohrungen (6) anschließen.

2. Dialyseschlauchsystem nach Anspruch 1, dadurch gekennzeichnet, daß die Schläuche (2) und (3) durch Lösungsmittelschweißen mit den Verbindungsstücken verbunden sind.

3. Dialyseschlauchsystem nach Anspruch 1 und 2, dadurch gekennzeichnet, daß die Verbindungsstücke (8) T-förmig gestaltet sind.

4. Dialyseschlauchsystem nach Anspruch 1, dadurch gekennzeichnet, daß das Verbindungsstück zwischen den Schläuchen als Deckel (24) und (25) für ein aus durchsichtigem Kunststoff geblasenes Hohlgefäß (19) gestaltet ist, wobei der Deckel (24) einen und der Deckel (25) mehrere Schlauchanschlüsse aufweist.

FIG.1

FIG.2

FIG.4

FIG.3

FIG.5

8

9

10

11

12

13

0033080

FIG. 9

2

24

FIG. 6a

14

FIG. 7a

17

14

18

15

16

FIG. 6

FIG. 7

19

FIG.8

FIG 10.a

FIG.10

FIG.10 b

FIG. 11

FIG. 12